# NEW EUROPEAN PATENT SPECIFICATION

(11) **EP 2 165 756 B2**
(45) Date of publication and mention of the opposition decision: **01.06.2016**
(45) Mention of the grant of the patent: 24.10.2012
(21) Application number: 08164753.9
(22) Date of filing: 19.09.2008
(51) Int. Cl.: B01J 8/02, B01D 53/86

(54) **Method for the decomposition of nitrous oxide in a gas stream**
Verfahren zur Dekomposition von Distickstoffmonoxid in einem Gasstrom
Procédé pour la décomposition d'oxyde nitreux dans un flux gazeux

(43) Date of publication of application: 24.03.2010
(73) Proprietor: Linde AG, 80331 München (DE)
(72) Inventor: Beran, Franz, 81476 München (DE); Hofmann, Karl-Heinz Dr., 82110 Germering (DE); Schödel, Nicole Dr., 81477 München (DE); Wenning, Ulrike Dr., 82049 Pullach (DE); Zander, Hans-Jörg Dr., 81479 München (DE); Schmehl, Wolfgang, 22525 Hamburg (DE); Wadham, Paul, Haslemere, Surrey GU27 3SW (GB)
(74) Representative: Dörries, Hans Ulrich

(56) References cited:
- WO-A-2006/059506
- US-A- 3 467 492
- US-A- 4 980 040
- US-A1- 2006 008 401
- US-B2- 7 235 222
- EK M ET AL: 'Mitig Adapt Strateg Glob Change', vol. 13, 2008, SPRINGER pages 809 - 818

## Description

The present invention relates to a method for the decomposition of nitrous oxide in a gas stream, in particular for the decomposition of nitrous oxide in the expiration air flow of a patient.

Gas mixtures of nitrous oxide and oxygen are widely used in medicine as analgesic and anesthetic gases, either in combination with or without other volatile and/or anesthetic drugs. Contrary to organic narcotics, nitrous oxide which is released during exhalation by the patient cannot simply be removed by physical methods like adsorption, absorption or separation. Therefore, exhaled residual nitrous oxide has usually been released into the operating room, delivery room, surgery, dental practice room, emergency room or ambulance or directly into the environment via an exhaust system. Nitrous oxide exposition over a long period of time, however, can be harmful to medical personnel even in very low concentrations in case of frequent exposition over many years (maximal allowable concentration in the U.S. according to the National Institute of Occupational Safety and Health (NIOSH): 25 ppm; "Maximale Arbeitsplatz-Konzentration" (MAK) according to the "Berufsgenossenschaftliches Institut fur Arbeitsschutz" in Germany: 100 ppm (or 50 ppm in some federal states of Germany)). Therefore, up to now, residual nitrous oxide generated in the treatment room is normally directly released into the environment where it contributes to the green house effect (about 300-times more potent in relation to carbon dioxide) and the destruction of the ozone layer.

For these reasons there is a strong need for decomposing nitrous oxide in waste anesthetic gas. US Patent No. 4,259,303 to Nakaji et al. (filed October 15, 1979) proposes a method for treating waste anesthetic gas by converting nitrous oxide to nitrogen and oxygen with a catalyst comprising one or more metal oxides selected from the group consisting of ferric oxide, cobalt oxide, cupric oxide, chromium oxide, manganese dioxide and nickel oxide.

US 2003/0185735 A1 to Hotta et al. (filed September 27, 2001) further develops and improves the known methods and apparatuses and additionally proposes mechanisms for removing water vapor and volatile organic analgesic, anesthetic, narcotic or other gases or vapors which could affect efficiency and lifetime of the decomposition catalyst. The document further discloses a decomposition reactor with a heat exchanger, and an electrical heater for raising the temperature of the nitrous oxide containing gas to be decomposed, which is operated when nitrous oxide is detected in the gas discharged from the decomposition reactor by a nitrous oxide monitoring means.

WO 2006/059506 A1 to Hotta et al. (filed November 15, 2005) slightly modifies the apparatus design of the decomposition reactor by integrating the electrical heater and specific baffles into a part of the decomposition reactor.

However, the apparatuses of Hotta et al. and other known constructions are very large devices designed for the decomposition of the waste gas stream of an entire hospital. They have to be installed stationarily and must be operated continuously under largely constant operating and nitrous oxide concentration conditions.

Up to now, there are no practical methods which can be used e.g. in a single operating room, delivery room, surgery, dental practice, emergency room or ambulance and can deal with sudden or intermittent occurrence and/or unsteady concentration of nitrous oxide that is typical in such operation conditions. One problem of the prior art apparatuses is that the catalyst in the nitrous oxide decomposition reactor cannot be operated under optimal reaction conditions (e.g., optimal nitrous oxide concentration and temperature range) during all phases of operation (i.e., during the start-up phase, high nitrous oxide concentrations, low nitrous oxide concentrations, or stand-by phase), resulting in a temporarily reduced degree of conversion (in particular during start-up and after stand-by phases) and a decreased life-span of the catalyst. In particular, after starting the apparatus, it takes between ten to thirty minutes before the catalytic reaction reaches its optimal degree of conversion. After interrupting the supply of nitrous oxide containing waste anesthetic gas the decomposition reactor of prior art apparatuses is cooling down and cannot be maintained in a ready-to-operate condition. Since nitrous oxide is often used for short time applications of several seconds or minutes or intermittently for smaller medical or surgical interventions or during delivery, prior art apparatuses are not appropriate for decomposing the nitrous oxide occurring under such operating conditions.

Furthermore said prior art devices are not appropriate for mobile indoor use, e.g., in operating rooms, delivery rooms, surgeries, dental practice rooms, emergency rooms or ambulances, where a compact design and the observance of high safety standards and other factors (e.g., high degrees of conversion of nitrous oxide at relatively low reaction temperatures, low energy consumption, low waste heat and/or low noise) are demanded.

It is therefore an object of the present invention to provide a method which overcomes the afore-stated problems of the prior art, i.e., a method that offers reliable decomposition of nitrous oxide under all occurring operating conditions and is also suitable for mobile indoor use, e.g., in operating rooms, delivery rooms, surgeries, dental practice rooms, emergency rooms or ambulances and under operating conditions where the occurrence and/or concentration of waste nitrous oxide to be decomposed is intermittent and/or temporally very unsteady.

This problem is solved by a method for the decomposition of nitrous oxide in a gas stream, in particular for the decomposition of nitrous oxide in the expiration air flow of a patient, according to claim 1.

The invention uses a device for the decomposition of nitrous oxide in an incoming gas stream, in particular for the decomposition of nitrous oxide in the expiration air flow of a patient, comprising a nitrous oxide decomposition reactor containing a nitrous oxide decomposition catalyst, preferably a noble metal catalyst; and a temperature controlling means for controlling the temperature in the nitrous oxide decomposition reactor.
The term "temperature controlling means" as used herein is intended to refer to means capable of, or suitable for, controlling the temperature in the nitrous oxide decomposition reactor by directly and/or indirectly influencing said temperature, e.g., by heating and/or limiting the temperature and/or channelling gas streams and/or utilising heat of the reaction, or by other means. Thus, the temperature controlling means are capable of, or suitable for, gaining or increasing control over the reaction temperature under different operating conditions. Likewise, "controlling the temperature in the nitrous oxide decomposition reactor" as used herein may be a direct and/or indirect influencing of said temperature, again e.g. by heating and/or limiting the temperature and/or channelling gas streams and/or utilising heat of the reaction, or by other means.

In the present invention the temperature controlling means are suitable for controlling the temperature in the nitrous oxide decomposition reactor independent of the concentration of nitrous oxide to be decomposed. With the help of these temperature controlling means, it is possible to further improve control over the reaction temperature under virtually all occurring operating conditions.

In another preferred embodiment of the present invention the temperature controlling means comprise at least one temperature sensor, preferably located in the vicinity of, or in the nitrous oxide decomposition reactor, in particular at least a temperature sensor upstream of the nitrous oxide decomposition reactor. By placing a sensor e.g. near the inlet of the decomposition reactor, it is possible to directly measure and control the temperature of the gas stream entering the decomposition reactor and thus to control e.g. a heat exchanger and/or an additional heater. In particular, it may be advantageous to place the temperature sensor in the gas stream, in a short distance to the catalyst fill, so that the sensor may be impacted by heat interchange with the gas stream and/or by radiant heat of the catalyst fill, which can improve response characteristics and quality of the measurement. Furthermore it may be desirable to mount a temperature sensor at the outlet of the decomposition reactor, e.g. for taking measures to limit the temperature increase due to the reaction heat of the nitrous oxide decomposition in the decomposition reactor in order to prevent the decomposition catalyst from overheating or exceeding the maximum desired or admissible reaction temperature. Additionally, by calculating the temperature increase in the decomposition reactor from the difference between the temperatures of the gas streams entering and leaving the decomposition reactor, the gas flow rate - under consideration of the thermal capacity of the decomposition reactor and the heat loss - the nitrous oxide quantity decomposed in the decomposition reactor and/or the nitrous oxide concentration in the gas stream can be rated. Moreover, it can be considered to install one or more temperature sensors inside the decomposition reactor to directly measure the temperature or temperature profile inside the decomposition reactor.

In another preferred embodiment of the present invention the device comprises a mixing means upstream of the nitrous oxide decomposition reactor for mixing the incoming gas stream with a diluting gas, preferably with ambient air, wherein the quantity of the diluting gas can be controlled preferably between 0% and 100%. This offers the possibility to reduce and limit the concentration of nitrous oxide entering the decomposition reactor by diluting the incoming nitrous oxide containing gas to a desired maximum nitrous oxide concentration of e.g. 10 mole% or less, preferably 6 mole% or less, in particular 2.5 mole% or less, in order to limit the temperature increase in the decomposition reactor, to prevent damaging of the catalyst and to ensure a high degree of conversion of the nitrous oxide. Furthermore, it is possible, e.g., during the start-up phases and/or the stand-by phases, to pass, e.g., exclusively diluting gas or ambient air (meaning 100% diluting gas or ambient air) through the decomposition reactor, which gas or air is heated by a heating means, preferably by an electrical heater in order to preheat and/or stabilize the decomposition reactor at a certain temperature level. The mixing or addition of diluting gas or ambient air in the mixing means may be achieved by bringing together the two gases by a branch connection, e.g., a T-piece or a Y-piece, - with or without a specific gas mixer. It will be understood that the mixing means may be part of, or may form the temperature controlling means according to the invention.

The mixing means in accordance with the present invention preferably further comprise a sensing means, wherein the quantity of the diluting gas added to the incoming gas stream is controlled depending on the measurement result of the sensing means. By such sensing means, for example, the dilution of the nitrous oxide containing incoming gas can be controlled more precisely. Such sensing means may comprise direct (e.g., electrochemical, (IR-)optical) or indirect (e.g., by estimating reaction heat or reaction enthalpy via measuring temperature increase in the decomposition reactor and calculating the converted nitrous oxide quantity) nitrous oxide sensors and/or flow rate sensors and/or one or more temperature sensors for gas streams.

In a preferred embodiment of the present invention the temperature controlling means comprise a heating means, preferably an electric heater. By such heater, it is possible to achieve and maintain a certain temperature level in the decomposition reactor e.g. during start-up phases, stand-by phases or phases with low nitrous oxide load.

In the present invention the temperature controlling means comprise a mechanism for heating up and/or stabilizing the temperature of the nitrous oxide decomposition reactor during start-up and/or stand-by and/or low-load phases by passing heated gas substantially free of nitrous oxide through the nitrous oxide decomposition reactor. By such heating up and/or stabilizing mechanism, it is possible to heat up the decomposition reactor to and/or stabilize it at a certain, predefined temperature level. Such temperature level may be identical to, or different from the standard reaction temperature; such temperature level can, thus, for example be the optimum reaction temperature, or a temperature at which at least a certain degree of nitrous oxide conversion, e.g., 25%, 50%, 60%, 70%, 80%, 85%, 90%, or even 95%, can be achieved, or a critical temperature level which allows starting of the reaction in the decomposition reactor, or a quick achievement of a required degree of conversion. The temperature level which is adjusted during start-up and/or stand-by phases may be adjusted e.g. according to the intended operating conditions, frequency and duration of dead times, occupational health or power efficiency requirements or legal regulations for medical equipment in hospitals and treatment rooms and/or energy consumption, waste heat or other consideration.

The temperature controlling means according to the present invention preferably comprise a mechanism for limiting the temperature in the nitrous oxide decomposition reactor. By providing means for limiting the temperature in the decomposition reactor it can be ensured that the decomposition reactor runs at a preferred operating temperature or within a preferred operating temperature range, thereby preventing possible damage to a decomposition catalyst that is easily damageable if exposed to higher nitrous oxide concentrations or during continuous long term operation. Thus, it is possible to apply sensitive catalysts, e.g., noble metal catalysts to the decomposition device of the invention, allowing higher degrees of conversion at lower reaction temperatures. Furthermore such limitation of the temperature in the decomposition reactor may be required for reasons of occupational safety and/or in order to comply with legal regulations for medical equipment in hospitals and treatment rooms.

In a preferred embodiment of the present invention the temperature controlling means comprise a heat exchanger with a temperature limiting mechanism controlling the rate of heat exchange of the heat exchanger, preferably comprising a bypass mechanism. A heat exchanger, exchanging heat between the gas volume flows entering and leaving the nitrous oxide decomposition reactor, increases energy efficiency of the device, reducing both, average and peak energy consumption and waste heat of the device, however, at the risk of exceeding a maximum allowable temperature in the decomposition reactor and of damaging the decomposition catalyst during phases with high nitrous oxide load. By providing a heat exchanger with a mechanism for limiting the rate of heat exchanged between the gas volume flows entering and leaving the nitrous oxide decomposition reactor, an upper temperature level in the decomposition reactor can be maintained. Thereby, exceeding a maximum allowable temperature in the decomposition reactor can be prevented, e.g., during long-term operation and/or at high nitrous oxide loads.

In another preferred embodiment of the present invention the device comprises feed-back means for feeding back the gas discharged from the nitrous oxide decomposition reactor - or a fraction thereof - into the inlet of the nitrous oxide decomposition reactor. By such feed-back means it is possible to completely or partially recycle the gas leaving the decomposition reactor, e.g. during start-up and/or low-load phases where the decomposition reactor is heated up or stabilized at a desired temperature, by making the gas circulate in a small loop through the electrical heater and the decomposition reactor. A further positive effect of such feed-back means is that during such phases, when the feed-back means is active, the above-mentioned mixing with ambient air can be reduced which helps saving energy and waste heat, increases decomposition rate and reduces noise during such phases. It is also possible to activate the feed-back means during high-load phases with high nitrous oxide concentrations in the gas to be decomposed and to use the feed-back of purified (or decomposed) gas leaving the decomposition reactor for the purpose of diluting the nitrous oxide containing gas to a desired maximum nitrous oxide concentration of e.g. 10 mole% or less, preferably 6 mole% or less, in particular 2.5 mole% or less, while at the same time replacing or reducing the addition of ambient air by the afore-described mixing means with the same positive effects as mentioned above. It will again be understood that the feed-back means may be part of, or may form the temperature controlling means according to the invention.

Said feed-back means preferably comprise a controlling means for controlling which quantity of the gas discharged from the nitrous oxide decomposition reactor is fed back into the inlet of the nitrous oxide decomposition reactor, depending on a temperature parameter and/or gas volume flow parameter and/or on a concentration and/or a quantity parameter of the nitrous oxide. By measuring one or more operation parameters, the feed-back means can be precisely controlled according to the afore-described technical teaching.

In a preferred embodiment of the present invention the device may further comprise flow smoothening means for the incoming gas stream, for reducing peaks or fluctuations in flow rate of the incoming gas stream, the means preferably being located upstream of the mixing means. By buffering the nitrous oxide containing gas exhaled by a patient e.g. in a rigid or elastic gas chamber, a hydraulic chamber or in a pressure tank, it is possible to reduce peaks or fluctuations in the flow rate, e.g. caused by breathing of the patient and thus to smoothen the flow-rate of the nitrous oxide containing gas further processed in the decomposition device. Thereby diluting the nitrous oxide containing gas to a desired nitrous oxide concentration by the downstream mixing means can be achieved more precisely and the flow rate - and thus the residence time in the decomposition reactor of the gas to be decomposed - can be optimized or a minimum residence time in the decomposition reactor of the gas to be decomposed can be observed.

The invention relates to a method for the decomposition of nitrous oxide in an incoming gas stream, in particular for the decomposition of nitrous oxide in the expiration air flow of a patient, in a nitrous oxide decomposition reactor, comprising the steps of controlling the temperature in the nitrous oxide decomposition reactor; and passing the incoming gas stream through the nitrous oxide decomposition reactor containing a nitrous oxide decomposition catalyst, preferably a noble metal catalyst.

In the present invention the controlling of the temperature in the nitrous oxide decomposition reactor is performed independently of the presence and/or concentration of nitrous oxide to be decomposed. By this approach, it is possible to further improve control over the reaction temperature under virtually all occurring operating conditions.

In another preferred embodiment of the present invention the temperature in the nitrous oxide decomposition reactor is controlled during a start-up phase - prior to passing the incoming gas stream through the nitrous oxide decomposition reactor -, during a stand-by phase, and/or during a decomposition phase. Also by this approach, it is possible to further improve control over the reaction temperature under virtually all occurring operating conditions.

In a preferred embodiment of the present invention the method comprises the step of sensing the temperature, preferably at least of the gas introduced into the nitrous oxide decomposition reactor. By placing a sensor e.g. near the inlet of the decomposition reactor, it is possible to directly measure and control the temperature of the gas stream entering the decomposition reactor and thus to control, e.g., a heat exchanger and/or an additional heater. In particular it may be advantageous to place the temperature sensor in the gas stream, in a short distance to the catalyst fill, so that the sensor may be impacted by heat interchange with the gas stream and by radiant heat of the catalyst fill, which can improve response characteristics and quality of the measurement. Furthermore it may be desirable to mount a temperature sensor at the outlet of the decomposition reactor, e.g. for taking measures to limit the temperature increase due to the reaction heat of the nitrous oxide decomposition in the decomposition reactor in order to prevent the decomposition catalyst form overheating or exceeding the maximum desired reaction temperature. Additionally, by calculating the temperature increase in the decomposition reactor from the difference between the temperatures of the gas streams entering and leaving the decomposition reactor, the gas flow rate - under consideration of the thermal capacity of the decomposition reactor and the heat loss - the nitrous oxide quantity decomposed in the decomposition reactor and/or the nitrous oxide concentration in the gas stream can be rated.

Moreover, it can be considered to install one or more temperature sensors inside the decomposition reactor to directly measure the temperature or temperature profile inside the decomposition reactor. It will be understood that the above step of sensing the temperature may be part of, or may represent, the step of controlling the temperature in the nitrous oxide decomposition reactor.

In yet another preferred embodiment of the present invention the method comprises the step of mixing the incoming gas stream with a diluting gas, preferably with ambient air, prior to passing the incoming gas stream through the nitrous oxide decomposition reactor, wherein the quantity of the diluting gas can be controlled preferably between 0% and 100%. This offers the possibility to reduce and limit the concentration of nitrous oxide entering the decomposition reactor by diluting the incoming nitrous oxide containing gas to a desired maximum nitrous oxide concentration of e.g. 10 mole% or less, preferably 6 mole% or less, in particular 2.5 mole% or less, in order to limit the temperature increase in the decomposition reactor, to prevent damaging of the catalyst and to ensure a high degree of conversion of the nitrous oxide. Furthermore, it is possible; e.g.; during the start-up phases and/or the stand-by phases, to pass, e.g., exclusively diluting gas or ambient air (meaning 100% diluting gas or ambient air) through the decomposition reactor, which gas or air is heated by a heating means, preferably an by electrical heater in order to preheat and/or stabilize the decomposition reactor at a certain temperature level. The mixing or addition of diluting gas or ambient air in the mixing means may be achieved by bringing together the two gases by a branch connection, e.g., a T-piece of a Y-piece, - with or without a specific gas mixer. Again, it will be understood that the above step of mixing may be part of, or may represent, the step of controlling the temperature in the nitrous oxide decomposition reactor.

In a preferred embodiment of the present invention the step of controlling the temperature may comprise, at least temporarily, additionally heating the gas entering the nitrous oxide decomposition reactor and/or heating the nitrous oxide decomposition reactor and/or an inlet pipe thereof, preferably by an electric heater, for starting and stabilizing the decomposition reaction in the nitrous oxide decomposition reactor. By such step of additionally heating, it is possible to achieve and maintain a certain temperature level in the decomposition reactor e.g. during start-up phases, stand-by phases or phases with low nitrous oxide load.

In the present invention the step of controlling the temperature also comprises stabilizing the temperature of the nitrous oxide decomposition reactor during start-up and/or stand-by and/or low-load phases by passing heated gas substantially free of nitrous oxide through the nitrous oxide decomposition reactor. By such stabilizing, it is possible to heat up the decomposition reactor to and/or stabilize it at a certain, predefined temperature level. Such temperature level may be identical to, or different from the standard reaction temperature; such temperature level can, thus, for example be the optimum reaction temperature or a temperature at which at least a certain degree of nitrous oxide conversion, e.g. 25%, 50%, 60%, 70%, 80%, 85%, 90%, or even 95%, can be achieved, or a critical temperature level which allows starting of the reaction in the decomposition reactor, or a quick achievement of a required degree of conversion. The temperature level which is adjusted during start-up and/or stand-by phases may be adjusted e.g. according to the intended operating conditions, frequency and duration of dead times, occupational health or power efficiency requirements or legal regulations for medical equipment in hospitals and treatment rooms and/or energy consumption, waste heat or other consideration.

In a preferred embodiment of the present invention the temperature of the nitrous oxide decomposition reactor during start-up and/or stand-by phases may be stabilized at a first target temperature, lower than a second target temperature during decomposition phase, wherein the first target temperature is preferably at least about 20°C, 30°C, 40°C, and in particular at least about 50°C, 75°C, 100°C, 125°C or even 150°C lower than the second target temperature. In this regard, the first target temperature should be construed as the temperature which is actively maintained e.g. by means of controlled heating whereas the second target temperature describes a preferred reaction temperature aimed atduring standard or average operating conditions during the decomposition phase. By choosing a lower preset temperature level during start-up and/or stand-by phases, power efficiency and waste heat during dead times of the decomposition device can be reduced.

In a preferred embodiment of the present invention the step of controlling the temperature further comprises limiting the temperature in the nitrous oxide decomposition reactor. By providing means for limiting the temperature in the decomposition reactor the decomposition reactor may be run at a preferred operating temperature or within a preferred operating temperature range, thus avoiding possible damage to a decomposition catalyst that can be easily damaged, if exposed to higher nitrous oxide concentrations or during continuous long term operation. Thus, it is possible to apply sensitive catalysts, e.g., noble metal catalysts for the decomposition devices, allowing higher degrees of conversion at lower reaction temperatures. Furthermore such limiting of the temperature in the decomposition reactor may be required for reasons of occupational safety and/or in order to comply with legal regulations for medical equipment in hospitals and treatment rooms.

In yet another preferred embodiment of the present invention the step of controlling temperature comprises exchanging heat between the gas volume flows entering and leaving the nitrous oxide decomposition reactor, wherein the rate of the heat exchanged between the gas volume flows entering and leaving the nitrous oxide decomposition reactor is preferably controlled by - at least temporarily - by passing the heat exchanger of a controlled fraction of any one of the gas volume flows entering and/or leaving the nitrous oxide decomposition reactor. A heat exchanger, exchanging heat between the gas volume flows entering and leaving the nitrous oxide decomposition reactor, increases energy efficiency of the device, thus reducing both, average and peak energy consumption and waste heat of the device, however, at the risk of exceeding a maximum allowable temperature in the decomposition reactor and of damaging the decomposition catalyst during phases with high nitrous oxide load. By providing a heat exchanger with a mechanism for limiting the rate of heat exchanged between the gas volume flows entering and leaving the nitrous oxide decomposition reactor, an upper temperature level in the decomposition reactor can be maintained. Thereby, exceeding a maximum allowable temperature in the decomposition reactor can be prevented, e.g., during long-term operation and/or at high nitrous oxide loads.

In a preferred embodiment of the present invention the method may comprise the step of feeding back at least temporarily the gas discharged from the nitrous oxide decomposition reactor - or a fraction thereof - into an inlet of the nitrous oxide decomposition reactor via a feed back loop. By such feeding-back, it is possible to completely or partially recycle the gas leaving the decomposition reactor, e.g. during start-up and/or low-load phases where the decomposition reactor is heated up or stabilized at a desired temperature, by making the gas circulate in a small loop through the electrical heater and the decomposition reactor. A further positive effect of such feeding-back is that during such phases, when the feeding-back is performed, the above-mentioned mixing with ambient air can be reduced which helps saving energy and waste heat, increases decomposition rate and reduces noise during such phases. It is also possible to perform the feeding-back during high-load phases with high nitrous oxide concentrations in the gas to be decomposed and to use the feed-back of purified gas leaving the decomposition reactor for the purpose of diluting the nitrous oxide containing gas to a desired maximum nitrous oxide concentration of e.g. 10 mole% or less, preferably 6 mole% or less, in particular 2.5 mole% or less, while at the same time replacing or reducing the addition of ambient air by the afore-described mixing with the same positive effects as mentioned above. Again, it will be understood that the above step of feeding back may be part of, or may represent, the step of controlling the temperature in the nitrous oxide decomposition reactor.

In another preferred embodimentof the present invention the quantity of the gas discharged from the nitrous oxide decomposition reactor fed back into the inlet of the nitrous oxide decomposition reactor is controlled depending on a temperature parameter and/or gas volume flow parameter and/or on a concentration and/or a quantity parameter of the nitrous oxide. By measuring one or more operation parameters, the feeding-back can be controlled precisely according to the afore-described technical teaching.

The method of the present invention preferably further comprises a step of smoothening fluctuations or peaks in the flow rate of the incoming gas stream, preferably prior to controlling the concentration of nitrous oxide in the incoming gas stream. By buffering the nitrous oxide containing gas exhaled by a patient e.g. in a rigid or elastic gas chamber, a hydraulic chamber or in a pressure tank, it is possible to reduce peaks or fluctuations in the flow rate, e.g. caused by breathing of the patient and thus to smoothen the flow-rate of the nitrous oxide containing gas further processed in the decomposition device. Thereby, diluting the nitrous oxide containing gas to a desired nitrous oxide concentration by the downstream mixing means can be achieved more precisely and the flow rate - and thus the residence time in the decomposition reactor of the gas to be decomposed - can be optimized or it can be ensured that a desired minimum residence time in the decomposition reactor of the gas to be decomposed is observed.

In a preferred embodiment the method or device of the present invention further comprises - at least temporarily - the step of or means for cooling the gas discharged from the nitrous oxide decomposition reactor, respectively. Thereby, it is possible to obey occupational safety considerations and/or legal regulations for medical equipment in hospitals and treatment rooms requiring the observance of a maximum outlet temperature for the purified gas discharged into the treatment room which may have to be lower than a certain temperature limit, e.g., 40°C, but also, e.g., 35°C, 45°C, 50°C, 55°C or 60°C.

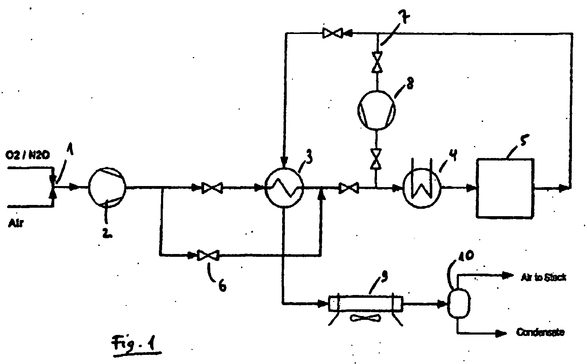

By way of example, one embodiment of the present invention is described below in greater detail together with the accompanying drawing, wherein
- Fig. 1: shows a schematic diagram of the operation of an apparatus for treating waste anesthetic gas according to an embodiment of the present invention.

Fig. 1, which shows a schematic diagram of the operation of an embodiment of an apparatus for treating waste anesthetic gas according to the method of the present invention, is described in the following in the direction of the main gas flow.

The anesthetic gas expired by a patient and containing nitrous oxide is captured from the expiration airflow of the patient, for instance by a respiratory mask. This gas containing a fraction of 0 to 70 mole% nitrous oxide may be withdrawn by suction by means of a blower, e.g., a blower being part of the respiratory mask equipment or one being located in the apparatus for treating waste anesthetic gas, or alternatively may be delivered with positive pressure from the patient's side (e.g. in case of positive pressure artificial respiration system with closed respiration mask) to the apparatus for treating waste anesthetic gas. In any case, the anesthetic nitrous oxide containing gas expired by the patient is either passively or actively conveyed to and entering the apparatus for treating waste anesthetic gas of the here-described embodiment (= incoming gas). Optionally (not shown), the incoming gas may be buffered in a rigid or elastic gas chamber, a hydraulic chamber or in a pressure tank in order to reduce peaks or fluctuations in the flow rate, e.g., caused by breathing of the patient and thus to smoothen the flow-rate of the exhaled nitrous oxide containing gas.

The incoming gas containing nitrous oxide may then optionally be analyzed regarding the parameters nitrous oxide concentration and volume per time unit or flow rate. During normal operation in a decomposition phase the incoming gas is subsequently mixed and diluted with ambient air in a mixing means 1 in order to reduce the maximum nitrous oxide concentration to e.g. 10 mole% or less, preferably to 6 mole% or less, in particular to 2.5 mole% or less. The mixing nitrous oxide containing gas with ambient air or addition of ambient air to the nitrous oxide containing gas in the mixing means 1 is achieved by bringing together the two gas streams by a branch connection. Additionally, a separate gas mixer may be provided (in this embodiment not shown).

Afterwards, the gas passes a negative pressure limiting valve (for protecting the patient from breathable air or gas mixture being withdrawn by a blower 2 of the apparatus for treating waste anesthetic gas; negative pressure limiting valve not shown) and said blower 2, which, in this case, serves simultaneously for generating a negative pressure for suction of both nitrous oxide containing gas and ambient air for diluting said nitrous oxide containing gas, and, additionally, to overcome the pressure drop of the system. Alternatively, separate means for conveying nitrous oxide containing gas on the one hand, and ambient air diluting said nitrous oxide containing gas on the other hand, may be provided.

A non-return valve (not shown) downstream of the blower 2 protects the patient from back-flux of hot gas. Also optionally (not shown) water vapor and organic analgesic, anesthetic, narcotic or other gases or vapors which could affect efficiency and lifetime of the decomposition catalyst are removed, preferably by methods of adsorption, absorption, separation or chemical reaction well known in the art.

The gas is then passed through a heat exchanger 3 and an electrical heater 4 prior to being supplied to the decomposition reactor 5 containing a decomposition catalyst. As decomposition catalyst any catalyst suitable for the decomposition of nitrous oxide may be used, preferably a noble metal catalyst, in particular a catalyst comprising one or more noble metals selected from the group consisting of Pd, Rh, Pt, Ru - especially preferred Pd and/or Rh - on a carrier like aluminium oxide, silicon oxide and/or zeolite.

The heat exchanger 3 is provided with a temperature controllable bypass 6 valve for the gas to be decomposed to short-circuit the heat exchanger. By means of said bypass 6 valve it is possible to limit the increase in temperature of the nitrous oxide containing gas entering the decomposition reactor 5 and thus also to control and limit the temperature inside the decomposition reactor 5 to a desired maximum temperature of, e.g., approximately 400°C or 450°C, which has to be observed and not to be exceeded despite the reaction heat of the exothermic decomposition reaction.

On the other hand, for example, when starting up the apparatus or during low-load phases with low nitrous oxide concentrations or during stand-by phases in the absence of a nitrous oxide containing gas stream entering the decomposition reactor 5, the heat exchanger 3 cannot sufficiently preheat the gas stream entering the decomposition reactor 5 to a value at which the decomposition reaction in the decomposition reactor 5 can start or continue, respectively. During such phases, which can be detected e.g. by a temperature sensor, the electrical heater 4 is activated in order to ensure a minimum temperature of the nitrous oxide containing gas (or any other gas, e.g., nitrous oxide-free gas or ambient air) entering the decomposition reactor 5 of, e.g., approximately 200°C or 250°C. The mechanisms during start-up and stand-by phases are described in further detail below.

The temperature of the gas streams entering and leaving the decomposition reactor 5 are measured in the vicinity of the decomposition reactor 5. The temperature sensors are placed in the gas stream, in a short distance to the catalyst fill, so that the temperature sensors are heated by both, the heat interchange with the gas stream and the radiant heat of the catalyst fill, in order to improve response characteristics and quality of the measurement. This offers the possibility for controlling the preheating of the gas entering the decomposition reactor 5, for observation of the upper temperature limit of the decomposition reactor, and for calculating or rating the nitrous oxide concentration and quantity via the temperature increase in the decomposition reactor caused by the reaction heat of the nitrous oxide decomposition, as mentioned above. During normal decomposition operation the intended reaction temperature is between about 100°C and about 500°C, preferably between about 150°C and about 450°C, in particular between about 200°C and about 400°C, wherein the maximum nitrous oxide concentration is 10 mole% or less, preferably 6 mole% or less, in particular 2.5 mole% or less and the pressure in the entire system ranges between atmospheric pressure and 1 bar (= 0.1 MPa) positive pressure.

Downstream of the decomposition reactor 5 the purified gas passes a branch connection with control valves for temporarily enabling the branching off of a controlled fraction of the purified gas (feed-back means 7) which subsequently passes another blower 8 and is fed back into the inlet pipe of the decomposition reactor 5 between the heat exchanger 3 and the electrical heater 4. By such feed-back means 7 it is possible to completely or partially recycle the gas leaving the decomposition reactor 5, e.g. during start-up and low-load phases where the decomposition reactor 5 is heated up or stabilized at a desired temperature, by making the gas circulate in a small loop through the electrical heater 4 and the decomposition reactor 5. A further positive effect of such feed-back 7 means is that during such phases, when the feed-back means is active, the above-mentioned mixing with ambient air in the mixing means can be reduced which helps saving energy and waste heat, increases decomposition rate and reduces noise during such phases. It is also possible to activate the feed-back means during high-load phases with high nitrous oxide concentrations in the gas to be decomposed and to use the feed-back of purified gas leaving the decomposition reactor 5 for the purpose of diluting the nitrous oxide containing gas to a desired maximum nitrous oxide concentration of e.g. 10 mole % or less, preferably 6 mole % or less, in particular 2.5 mole% or less, while at the same time replacing or reducing the addition of ambient air by the afore-described mixing means 2 with the same positive effects as mentioned above.

The remaining purified gas which is notfed back to the decomposition reactor 5 is then passed again through the above-mentioned heat exchanger 3 thereby transferring heat energy to the gas entering the decomposition reactor 5. Thus the nitrous oxide containing gas is heated up without additional energy consumption and waste heat while the purified gas is, at the same time, cooled down.

Finally the purified gas may be further cooled down by a cooling means or aftercooler 9 to ensure a maximum release temperature of 40°C which is normally required for indoor use in a hospital environment for safety reasons. This is preferably achieved by a flow-through cooling unit. Alternatively or additionally the purified gas can be cooled down by mixing the purified gas leaving the heat exchanger or aftercooler 9 at a temperature above 40°C with room temperature ambient air (not shown). In order to control this cooling process, the temperature of the purified gas may be measured before and after the cooling means 9 for controlling active heat dissipation of the cooling unit via forced cooling by a blower (not shown) and/or for calculating the quantity of ambient air required for being admixed to the purified gas. Finally the purified and cooled down gas stream is passed through a condensate drum 10 and released into the room or into an exhaust system.

In operating rooms, delivery rooms, surgeries, dental practices, emergency rooms or ambulances nitrous oxide is often used for short time applications of several seconds or minutes or intermittently for smaller medical or surgical interventions or during delivery so that the apparatus for treating waste anesthetic gas has to deal with a sudden or intermittent occurrence and/or unsteady concentration of nitrous oxide. At least five phases may be distinguished, that may occur during such operating conditions:
1. start-up phase when the apparatus is not yet ready for decomposition, prior to nitrous oxide being passed through the apparatus; during such phase, the decomposition reactor is preheated to operating temperature or to a certain temperature level, e.g. sufficient for starting the decomposition reaction 5 or for observing certain specifications, as explained above, until the apparatus is ready for operation.
2. normal decomposition phase, when the apparatus is operating normally with ordinary nitrous oxide loads.
3. high-load operation, occurring during certain phases of anesthesia or certain physiological conditions of a patient; during such phases, where the nitrous oxide quantity per time unit is considerably increased, there is a risk of overheating the decomposition reactor 5, since with rising nitrous oxide quantities the reaction heat increases, resulting in an elevated heat transfer to the gas entering the decomposition reactor 5 in the heat exchanger 3 which additionally boosts the heat in the decomposition reactor 5. During such phases means for limiting temperature (e.g. a bypass pathway 6 for the heat exchanger 3) are important in order not to exceed an upper temperature limit in the decomposition reactor 5 for reasons of operational safety and/or lifetime of the decomposition catalyst.
4. low-load operation when the nitrous oxide concentration falls below a limit where the reaction is on the verge of terminating, due to insufficient reaction heat and, thus, insufficient preheating of the gas entering the decomposition reactor 5; during such phases additional heating of the gas entering the decomposition reactor 5 with an electrical heater 4, which is preferably controlled by one or more of the afore-mentioned temperature sensors, is required.
5. stand-by phase where no nitrous oxide containing gas is to be decomposed; during such phases the temperature in the decomposition reactor 5 has to be maintained at a certain level, as explained above, to preserve readiness of operation of the apparatus.

During phase (1) and phase (5), where no nitrous oxide containing gas passes the decomposition reactor 5, there are several options for achieving and maintaining readiness of operation of the nitrous oxide decomposition apparatus: passing substantially nitrous oxide free gas, which is preheated, through the decomposition reactor 5 (mixing means 2 e.g. setto 100% ambient air) in order to achieve operating temperature in the decomposition reactor 5 (this measure can e.g. also be combined with direct heating of the decomposition reactor 5); or, feeding-back gas discharged from the decomposition reactor via an electrical heater 4 again to the decomposition reactor 5, as explained above. Equal measures as mentioned above are also suitable for stabilizing the temperature in the decomposition reactor 5 during phase (4).

By such heating up and/or stabilizing mechanisms, it is possible to heat up to and/or stabilize the decomposition reactor 5 at a certain, predefined temperature level. Such temperature level may be identical to, or different from the standard reaction temperature; such temperature level can, thus, for example be the optimum reaction temperature or a temperature at which at least a certain degree of conversion, e.g. 25%, 50%, 60%, 70%, 80%, 85%, 90%, or even 95%, can be achieved, or a critical temperature level which allows starting of the reaction in the decomposition reactor 5, or a quick achievement of a required degree of conversion or other criteria as explained above. The temperature level which is adjusted during start-up and/or stand-by phase may be adjusted e.g. according to the intended operating conditions, frequency and duration of dead times, occupational health or power efficiency requirements or legal regulations for medical equipment in hospitals and treatment rooms and/or energy consumption, waste heat or other consideration. In compliance with such considerations, the temperature of the nitrous oxide decomposition reactor 5 during start-up and/or stand-by phases is stabilized at a target temperature which is preferably at least about 20°C, 30°C, 40°C, and in particular at least about 50°C, 75°C, 100°C, 125°C or even 150°C lower than the preferred reaction temperature during decomposition phase.

The apparatus for treating waste anesthetic gas according the afore-described embodiment is intended for mobile indoor use in an operating room, a delivery room, a surgery, a dental practice, an emergency room or an ambulance. It is, however, also possible to construct larger devices which are operated in separate rooms of a hospital as central purifying units disposing of e.g. the waste anesthetic gas of several or all operating and delivery rooms of the hospital. Furthermore it is possible to temporarily store the waste anesthetic gas e.g. in pressure tanks and to transport the gas in said pressure tanks to a central decomposition unit, offering the advantage of uniform operation, better utilization and efficiency with less start-up and stand-by phases of the decomposition device. Since the apparatus and method for the decomposition of nitrous oxide according to the invention, including the afore-described specific embodiment, is also suitable for low-load operation with nitrous oxide concentrations in the incoming gas stream in a range below, and even far below, 1 mole %, it is also possible to operate such apparatus for removing waste anesthetic gas from the ambient air of a delivery room, containing typically a nitrous oxide concentration of tens to hundreds of ppm, where the mother-to-be is often moving around in the room while exhaling the waste anesthetic gas to ambient air without a respiratory mask. In such case the step of diluting the nitrous oxide containing gas described above can be omitted.

### List of reference signs

- 1: mixing means
- 2: blower
- 3: heat exchanger
- 4: electrical heater
- 5: decomposition reactor with decomposition catalyst
- 6: bypass valve
- 7: feed-back means
- 8: blower
- 9: aftercooler
- 10: condensate drum

## Claims

1. Method for the decomposition of nitrous oxide in an incoming gas stream, in particular for the decomposition of nitrous oxide in the expiration air flow of a patient, in a nitrous oxide decomposition reactor, comprising the steps of:
controlling the temperature in the nitrous oxide decomposition reactor; and
passing the incoming gas stream through the nitrous oxide decomposition reactor containing a nitrous oxide decomposition catalyst, preferably a noble metal catalyst,
wherein the controlling of the temperature in the nitrous oxide decomposition reactor is independent of the concentration of nitrous oxide to be decomposed, **characterized in that**
the step of controlling the temperature also comprises heating up and stabilizing the temperature of the nitrous oxide decomposition reactor during stand-by phases by passing heated gas substantially free of nitrous oxide through the nitrous oxide decomposition reactor.

2. The method of claim 1, wherein the temperature in the nitrous oxide decomposition reactor is controlled during a start-up phase - prior to passing the incoming gas stream through the nitrous oxide decomposition reactor-, during a stand-by phase, or during a decomposition phase.

3. The method of claim 1 or 2, comprising the step of sensing the temperature, preferably at least of the gas introduced into the nitrous oxide decomposition reactor.

4. The method of anyone of the preceding claims, comprising the step of mixing the incoming gas stream with a diluting gas, preferably ambient air, prior to passing the incoming gas stream through the nitrous oxide decomposition reactor, wherein the quantity of the diluting gas can be controlled preferably between 0% and 100%.

5. The method of anyone of the preceding claims, wherein the step of controlling the temperature comprises, at least temporarily, additionally heating the gas entering the nitrous oxide decomposition reactor and/or heating the nitrous oxide decomposition reactor and/or an inlet pipe thereof, preferably by an electric heater, for starting and stabilizing the decomposition reaction in the nitrous oxide decomposition reactor.

6. The method of anyone of the preceding claims, wherein the temperature of the nitrous oxide decomposition reactor during start-up and/or stand-by phases is stabilized at a first target temperature, lower than a second target temperature during decomposition phase, wherein the first target temperature is preferably at least about 20°C, 30°C, 40°C, and in particular at least about 50°C, 75°C, 100°C, 125°C or even 150°C lower than the second target temperature.

7. The method of claim 6, wherein the second target temperature is between about 100°C and about 500°C, preferably between about 150°C and about 450°C, in particular between about 200°C and about 400°C or between about 400°C and 450°C.

8. The method of anyone of the preceding claims, wherein the step of controlling the temperature further comprises limiting the temperature in the nitrous oxide decomposition reactor.

9. The method of anyone of the preceding claims, wherein the step of controlling temperature comprises exchanging heat between the gas volume flows entering and leaving the nitrous oxide decomposition reactor, wherein the rate of the heat exchanged between the gas volume flows entering and leaving the nitrous oxide decomposition reactor is preferably controlled by - at least temporarily - bypassing the heat exchanger of a controlled fraction of anyone of the gas volume flows entering and/or leaving the nitrous oxide decomposition reactor.

10. The method of anyone of the preceding claims, comprising the step of feeding back at least temporarily the gas - or a fraction thereof - discharged from the nitrous oxide decomposition reactor into an inlet of the nitrous oxide decomposition reactor via a feed back loop.

11. The method of anyone of the preceding claims, wherein the quantity of the gas discharged from the nitrous oxide decomposition reactor fed back into the inlet of the nitrous oxide decomposition reactor is controlled depending on a temperature parameter and/or gas volume flow parameter and/or on a concentration and/or a quantity parameter of the nitrous oxide.

12. The method of anyone of the preceding claims, further comprising a step of smoothening fluctuations or peaks in the flow rate of the incoming gas stream, preferably prior to controlling the concentration of nitrous oxide in the incoming gas stream.

13. The method of anyone of the preceding claims, further comprising - at least temporarily - the step of or a means for cooling the gas discharged from the nitrous oxide decomposition reactor, respectively.

## Patentansprüche

1. Verfahren für die Zersetzung von Stickstoffoxid in einem ankommenden Gasstrom, insbesondere für die Zersetzung von Stickstoffoxid im Ausatmungsluftstrom eines Patienten, in einem Stickstoffoxidzersetzungsreaktor, die Schritte umfassend:
Steuern der Temperatur im Stickstoffoxidzersetzungsreaktor; und
Leiten des ankommenden Gasstroms durch den Stickstoffoxidzersetzungsreaktor, welcher einen Stickstoffoxidzersetzungskatalysator beinhaltet, bevorzugt einen Edelmetallkatalysator,
wobei die Steuerung der Temperatur im Stickstoffoxidzersetzungsreaktor unabhängig von der zu zersetzenden Konzentration des Stickstoffoxids ist, **dadurch gekennzeichnet, dass**
der Schritt der Steuerung der Temperatur auch das Beheizen und Stabilisieren der Temperatur des Stickstoffoxidzersetzungsreaktors während Bereitschaftsphasen umfasst, indem beheiztes Gas, welches im Wesentlichen frei von Stickstoffoxid ist, durch den Stickstoffoxidzersetzungsreaktor geleitet wird.

2. Verfahren nach Anspruch 1, wobei die Temperatur in dem
Stickstoffoxidzersetzungsreaktor während einer Anlaufphase - vor dem Leiten des ankommenden Gasstroms durch den Stickstoffoxidzersetzungsreaktor -, während einer Bereitschaftsphase oder während einer Zersetzungsphase gesteuert wird.

3. Verfahren nach Anspruch 1 oder 2, umfassend den Schritt des Abfühlens der Temperatur, bevorzugt zumindest des Gases, das in den Stickstoffoxidzersetzungsreaktor eingeführt wird.

4. Verfahren nach einem der vorhergehenden Ansprüche, umfassend den Schritt des Mischens des ankommenden Gasstroms mit einem Verdünnungsgas, bevorzugt mit Umgebungsluft, vor dem Leiten des ankommenden Gasstroms durch den Stickstoffoxidzersetzungsreaktor, wobei die Menge des Verdünnungsgases bevorzugt zwischen 0% und 100% gesteuert werden kann.

5. Verfahren nach einem der vorhergehenden Ansprüche, wobei der Schritt der Steuerung der Temperatur zumindest vorübergehend ein zusätzliches Beheizen des Gases, das in den Stickstoffoxidzersetzungsreaktor eintritt und/oder ein Beheizen des Stickstoffoxidzersetzungsreaktors und/oder einer Zuleitung davon, bevorzugt durch eine elektrische Heizung, zum Starten und Stabilisieren der Zersetzungsreaktion in dem Stickstoffoxidzersetzungsreaktor umfasst.

6. Verfahren nach einem der vorhergehenden Ansprüche, wobei die Temperatur des Stickstoffoxidzersetzungsreaktors während der Anlauf- und/oder Bereitschaftsphasen bei einer ersten Zieltemperatur, welche niedriger als eine zweite Zieltemperatur während der Zersetzungsphase ist, stabilisiert wird, wobei die erste Zieltemperatur bevorzugt mindestens etwa 20 °C, 30 °C, 40 °C und insbesondere mindestens etwa 50 °C, 75 °C, 100 °C, 125 °C oder sogar 150 °C niedriger als die zweite Zieltemperatur ist.

7. Verfahren nach Anspruch 6, wobei die zweite Zieltemperatur zwischen etwa 100 °C und etwa 500 °C, bevorzugt zwischen etwa 150 °C und etwa 450 °C, insbesondere zwischen etwa 200 °C und etwa 400 °C oder zwischen etwa 400 °C und 450 °C liegt.

8. Verfahren nach einem der vorhergehenden Ansprüche, wobei der Schritt der Steuerung der Temperatur weiterhin das Begrenzen der Temperatur in dem Stickstoffoxidzersetzungsreaktor umfasst.

9. Verfahren nach einem der vorhergehenden Ansprüche, wobei der Schritt der Steuerung der Temperatur den Austausch von Wärme zwischen den die in den Stickstoffoxidzersetzungsreaktor eintretenden und ihn verlassenden Gasvolumenströmen umfasst, wobei das Verhältnis der Wärme, welche zwischen den die in den Stickstoffoxidzersetzungsreaktor eintretenden und ihn verlassenden Gasvolumenströmen ausgetauscht wird, bevorzugt- mindestens vorübergehend - durch Umgehen des Wärmetauschers um einen kontrollierten Anteil irgendeiner der Gasvolumenströme, die in den Stickstoffoxidzersetzungsreaktor eintreten und/oder ihn verlassen, gesteuert wird.

10. Verfahren nach einem der vorhergehenden Ansprüche, umfassend den Schritt des zumindest vorübergehenden Zurückleitens des Gases - oder eines Anteils davon -, das von dem Stickstoffoxidzersetzungsreaktor in einen Einlass des Stickstoffoxidzersetzungsreaktors über eine Rückleitungsschleife abgeführt wird.

11. Verfahren nach einem der vorhergehenden Ansprüche, wobei die Menge des von dem Stickstoffoxidzersetzungsreaktor abgeführten und in den Einlass des Stickstoffoxidzersetzungsreaktors zurückgeleiteten Gases abhängig von einem Temperaturparameter und/oder Gasvolumenströmungsparameter und/oder einer Konzentration und/oder einem Mengenparameter des Stickstoffoxids gesteuert wird.

12. Verfahren nach einem der vorhergehenden Ansprüche, weiter umfassend den Schritt des Glättens von Schwankungen oder Spitzenwerten in der Flussrate des eintretenden Gasstroms, bevorzugt vor der Steuerung der Konzentration von Stickstoffoxid in dem eintretenden Gasstrom.

13. Verfahren nach einem der vorhergehenden Ansprüche, weiter umfassend - zumindest vorübergehend - jeweils den Schritt des oder ein Mittel zum Kühlen des von dem Stickstoffoxidzersetzungsreaktor abgeführten Gases.

## Revendications

1. Procédé pour la décomposition d'oxyde nitreux dans un courant de gaz entrant, en particulier pour la décomposition d'oxyde nitreux dans l'écoulement d'air expiré d'un patient, dans un réacteur de décomposition d'oxyde nitreux, comprenant les étapes consistant à :
contrôler la température dans le réacteur de décomposition d'oxyde nitreux ; et
faire passer le courant de gaz entrant à travers réacteur de décomposition d'oxyde nitreux contenant un catalyseur de décomposition d'oxyde nitreux, de préférence un catalyseur à base de métal noble,
dans lequel le contrôle de la température dans le réacteur de décomposition d'oxyde nitreux est indépendant de la concentration en oxyde nitreux à décomposer, **caractérisé en ce que**
l'étape de contrôler de la température comprend également le chauffage et la stabilisation de la température du réacteur de décomposition d'oxyde nitreux au cours de phases d'attente en faisant passer un gaz chauffé sensiblement exempt d'oxyde nitreux à travers le réacteur de décomposition d'oxyde nitreux.

2. Procédé selon la revendication 1, dans lequel la température dans le réacteur de décomposition d'oxyde nitreux est contrôlée au cours d'une phase de démarrage, avant de faire passer le courant de gaz entrant dans le réacteur de décomposition d'oxyde nitreux, au cours d'une phase d'attente, ou au cours d'une phase de décomposition.

3. Procédé selon la revendication 1 ou 2, comprenant l'étape de détection de la température, de préférence au moins du gaz introduit dans le réacteur de décomposition d'oxyde nitreux.

4. Procédé selon l'une quelconque des revendications précédentes, comprenant l'étape consistant à mélanger le courant de gaz entrant avec un gaz de dilution, de préférence de l'air ambiant, avant de faire passer le courant de gaz entrant à travers le réacteur de décomposition d'oxyde nitreux, dans lequel la quantité du gaz de dilution peut être contrôlée de préférence entre 0 % et 100 %.

5. Procédé selon l'une quelconque des revendications précédentes, dans lequel l'étape consistant à contrôler la température comprend en outre, au moins temporairement, le chauffage du gaz entrant dans le réacteur de décomposition d'oxyde nitreux et/ou le chauffage du réacteur de décomposition d'oxyde nitreux et/ou d'une conduite d'entrée de celui-ci, de préférence par un dispositif de chauffage électrique, pour déclencher et stabiliser la réaction de décomposition dans le réacteur de décomposition d'oxyde nitreux.

6. Procédé selon l'une quelconque des revendications précédentes, dans lequel la température du réacteur de décomposition d'oxyde nitreux pendant les phases de démarrage et/ou d'attente est stabilisée à une première température cible, inférieure à une seconde température cible pendant la phase de décomposition, dans lequel la première température cible est de préférence au moins environ 20°C, 30 °C, 40°C, et en particulier au moins environ 50°C, 75°C, 100°C, 125°C, ou même 150°C inférieure à la seconde température cible.

7. Procédé selon la revendication 6, dans lequel la seconde température cible est comprise entre environ 100°C et environ 500 °C, de préférence entre environ 150 °C et environ 450 °C, en particulier entre environ 200°C et environ 400 °C ou entre environ 400°C et 450 °C.

8. Procédé selon l'une quelconque des revendications précédentes, dans lequel l'étape de contrôle de la température comprend en outre à limiter la température dans le réacteur de décomposition d'oxyde nitreux.

9. Procédé selon l'une quelconque des revendications précédentes, dans lequel l'étape de contrôle de la température comprend l'échange de chaleur entre les écoulements de volume de gaz entrant et sortant du réacteur de décomposition d'oxyde nitreux, dans lequel le débit de chaleur échangée entre les écoulements de volume de gaz entrant et sortant du réacteur de décomposition d'oxyde nitreux est de préférence contrôlé par, au moins temporairement, le contournement de l'échangeur de chaleur d'une fraction contrôlée selon l'un quelconque des écoulements de volume de gaz entrant et/ou sortant du réacteur de décomposition d'oxyde nitreux.

10. Procédé selon l'une quelconque des revendications précédentes, comprenant l'étape consistant à renvoyer au moins temporairement le gaz, ou une fraction de celui-ci, évacué du réacteur de décomposition d'oxyde nitreux vers une entrée du réacteur de décomposition d'oxyde nitreux par l'intermédiaire d'une boucle de réintroduction.

11. Procédé selon l'une quelconque des revendications précédentes, dans lequel la quantité du gaz évacué du réacteur de décomposition d'oxyde nitreux réintroduit au niveau de l'entrée du réacteur de décomposition d'oxyde nitreux est contrôlée en fonction d'un paramètre de température et/ou d'un paramètre d'écoulement de volume de gaz et/ou d'une concentration et/ou d'un paramètre de quantité de l'oxyde nitreux.

12. Procédé selon l'une quelconque des revendications précédentes, comprenant en outre une étape consistant à lisser des fluctuations ou des pics au niveau du débit du courant de gaz entrant, de préférence avant le contrôle de la concentration en oxyde nitreux dans le courant de gaz entrant.

13. Procédé selon l'une quelconque des revendications précédentes, comprenant en outre, au moins temporairement, l'étape consistant à ou un moyen pour refroidir le gaz évacué du réacteur de décomposition d'oxyde nitreux, respectivement.
